# FASCICULE DE BREVET EUROPEEN

(11) **EP 4 173 566 B1**
(45) Date de publication et mention de la délivrance du brevet: **18.06.2025**
(21) Numéro de dépôt: 22198425.5
(22) Date de dépôt: 28.09.2022
(51) Int. Cl.: A61B 5/11, A61B 5/08, A61B 5/00, A61B 5/0205, A61B 5/024, A61B 5/113, A47C 23/06, A47C 19/02

(54) **APPAREIL DE DETECTION POUR LITERIE**
BETTZEUG-DETEKTIONSVORRICHTUNG
SENSING APPARATUS FOR BEDDING

(30) Priorité: 28.10.2021 FR 2111494
(43) Date de publication de la demande: 03.05.2023
(73) Titulaire: Withings, 92130 Issy-les-Moulineaux (FR)
(72) Inventeur: Clerc, Samuel, 92130 Issy-les-Moulineaux (FR); Vercamer, Vincent, 92130 Issy-les-Moulineaux (FR); Cuniasse, Pierre-Antoine, 92130 Issy-les-Moulineaux (FR)
(74) Mandataire: Withings IP

(56) Documents cités:
- EP-A1- 3 828 598
- WO-A1-2021/008737
- US-A- 4 320 766

## Description

### Domaine technique

L'invention se rapporte aux systèmes et appareils que l'on peut placer dans une literie pour collecter des données sur le sommeil du ou des utilisateurs de la literie en question.

### Contexte et Art Antérieur

Le document EP2873368 décrit un dispositif à mettre sous ou sur le matelas, afin de détecter par ballistocardiographie au moins les mouvements, les rythmes cardiaque et respiratoire (par détection des mouvements voire micro-mouvements) d'un individu reposant sur le matelas, en vue de surveiller le sommeil de l'individu.

Le document EP3508187 décrit un dispositif similaire, avec une poche gonflable, qui vise à mitiger des difficultés de sensibilité liée à la rigidité du matelas. Pour pallier cette difficulté, il a notamment été proposé de mettre une structure rigide distincte du matelas pour distribuer les forces variables sur le dispositif. Cette structure est disposée entre le matelas et la poche gonflable. Une telle structure n'est pas commode à utiliser et baisse la manipulabilité du dispositif (emballage, déballage, stockage, etc.).

Les documents WO 2021/008737 et EP3828598 décrivent également des dispositifs similaires.

### Exposé de l'invention

Néanmoins, les inventeurs se sont aperçus que les dispositifs de l'art antérieur pouvaient présenter des performances qui dépendaient toujours du type de literie. En effet, ils ont découvert que la nature du sommier, sur lequel repose le dispositif, pouvait impacter les performances.

Il est ainsi proposé un appareil d'acquisition de données physiologique d'un être vivant (généralement un humain) présentant une sensibilité moindre au sommier.

Selon un aspect, il est proposé ici un appareil d'acquisition de données physiologiques d'un être vivant, l'appareil étant configuré pour être installé dans une literie, l'appareil d'acquisition comprenant une portion d'acquisition qui comprend :
- un capteur destiné à capter des ondes mécaniques transitant dans la literie (typiquement générées par l'être vivant),
- un dispositif de support comprenant au moins un panneau rigide, positionné pour supporter le capteur.

Le dispositif de support est configuré pour, dans une configuration d'utilisation de l'appareil d'acquisition dans la literie, être en appui sur des éléments de sommier de sorte que le dispositif de support soit interposé entre les éléments de sommier et le capteur. Les éléments de sommier peuvent être discontinus, comme par exemple des lattes.

On fournit ainsi un appui substantiellement continu au capteur recevant les ondes mécaniques générées par un utilisateur au travers de la literie. Les ondes mécaniques proviennent notamment des battements du cœurs de l'utilisateur, de sa respiration ou des autres mouvements de son corps.

Grâce à ces dispositions, on évite que le capteur ne descende entre les lattes et ne s'y coince. On évite aussi que le capteur ne s'affaisse localement et perte en sensibilité de détection des ondes mécaniques (surtout celles plus faibles du cœur et de la respiration).

Le capteur peut être de différentes technologies, à savoir un capteur de mouvement et/ou de pression, comme un capteur pneumatique, piézoélectrique ou de déformation, comme une jauge de déformation, ou bien encore une autre technologie. Le dispositif de support, placé sous le capteur, fournit un support adéquat pour maximiser l'efficacité, la sensibilité et la précision du capteur.

Sous le terme 'rigide' à propos du panneau rigide, il faut comprendre par exemple que la structure qui compose le panneau présente une rigidité équivalente à un matériau ayant un module de Young supérieur à 1 GPa. On note que le ou les panneau(s) présente(nt) toutefois une certaine souplesse pour pouvoir se conformer à la géométrie générale des lattes du sommier qui elles-mêmes présentent une souplesse en réaction à un poids subi (i.e. à un individu reposant sur le matelas).

On remarque que l'épaisseur de la portion d'acquisition est faible par exemple inférieure à 10 mm voire inférieure à 5 mm. La portion d'acquisition de l'appareil peut ainsi être facilement inséré et interposé entre le matelas et le sommier, sans faire sensiblement saillie dans le matelas, ni d'abimer ou de déformer durablement le matelas.

Il faut également noter que l'appareil d'acquisition fonctionne correctement avec un matelas d'une épaisseur quelconque par-dessus.

L'appareil d'acquisition peut intégrer une unité électronique, pour mettre en forme et/ou traiter des données brutes délivrées par le capteur. Ceci est réalisé sans surépaisseur substantielle.

La portion d'acquisition s'étend selon une direction longitudinale (X) et le dispositif de support comprend une pluralité de panneaux rigides agencés les uns à côté des autres selon l'axe longitudinal de la portion d'acquisition. Deux panneaux adjacents sont reliés entre eux par une zone de pliage autorisant une pliure du dispositif de support. La pliure est d'axe parallèle à une direction orthogonale à la direction longitudinale (X) (appelée direction transversale (Y)).

Moyennant quoi, grâce aux zones de pliage, il est possible de replier la portion d'acquisition et le dispositif de support en vue du rangement de l'appareil. Les zones de pliage permettent aussi accessoirement à la portion d'acquisition de se conformer à la géométrie du sommier, et de suivre l'élasticité des lattes sachant que les lattes ne fléchissent pas uniformément, e.g. en fonction de la répartition du poids appliqué sur le matelas.

Sous le terme 'à côté', il faut comprendre ici : adjacent ou légèrement à distance. Autrement dit, les panneaux se suivent selon l'axe longitudinal, en se touchant ou en étant séparés par un espace.

On remarque que pour désigner les éléments du dispositif de support, au lieu du terme 'panneau' on pourrait utiliser le terme 'plaque' ou 'plaquette'. Le panneau peut être plein ou ajouré (par exemple en treillis).

Selon une option, chacune des zones de pliage peut être formée comme une zone de charnière, par exemple parallèle la direction transversale (Y). On peut obtenir ainsi une forme pliée compacte, avec plusieurs zones de charnières, soit sous forme d'un enroulement, soit sous forme d'un accordéon.

Selon une option, les panneaux sont intégrés à une gaine, par exemple une gaine souple, qui s'étend le long de la direction longitudinale (X), les zones de la gaine souple qui sont dépourvues de panneaux peuvent former des zones de pliage. La gaine souple est un moyen simple de maintenir en place les panneaux les uns par rapport aux autres et par rapport au capteur. Le maintien en place doit être compris hors mouvement de pliage. La gaine souple peut être faite d'un matériau continu ou discontinu, e.g. ajourée à la façon d'un filet.

Selon une option, la gaine souple forme une pluralité de logements, les panneaux étant insérés dans les logements, par exemple un panneau par logement. Les logements sont agencés à la suite les uns des autres le long de la direction longitudinale (X). Entre deux logements se trouve une zone de jonction, qui forme zone de pliage comme décrite précédemment. On forme ainsi une solution simple et facile de mise en œuvre pour assembler les panneaux dans la gaine souple. Il peut être prévu une ouverture légèrement déformable dans le tissu de la gaine pour autoriser l'insertion du panneau rigide après quoi il est retenu à l'intérieur du logement naturellement notamment par l'élasticité du tissu. Alternativement, la gaine est cousue ou scellée de sorte que les panneaux ne sont plus accessibles. La gaine permet d'éviter à l'utilisateur d'être au contact des panneaux rigides (risque de coupure ou autre). Elle procure en outre un toucher plus confortable.

Selon une option, chacun des panneaux présente une forme générale rectangulaire et présente une épaisseur constante, par exemple inférieure à 3mm, voire 2mm, voire 1mm. L'épaisseur peut dépendre du matériau. Chaque panneau est léger et peu coûteux à fabriquer.

Selon une option, il peut y avoir plusieurs panneaux dans un logement. Cela augmente encore les possibilités d'enroulement ou de pliage pour transformer l'appareil d'acquisition en un élément peu encombrant.

Selon une option, le capteur présente une largeur de capteur (LY) le long d'une direction transversale (Y) et chaque panneau rigide s'étend, le long de la direction transversale, sur substantiellement toute la largeur du capteur. Par substantiellement, on entend au moins 80%, voire 90%, de la largeur de capteur du capteur.

Selon une option, l'appareil d'acquisition peut comprendre en outre une housse enveloppant au moins la portion d'acquisition. La housse protège ainsi le capteur.

Selon une option, la housse est amovible. On peut ainsi procéder au lavage ou nettoyage de la housse si nécessaire.

Selon une option, la housse est distincte de la gaine souple. La gaine souple permet de maintenir l'assemblage des panneaux rigides, et la housse vient recouvrir l'ensemble qui inclut aussi le capteur.

Selon une option, une partie de la housse fait office de gaine souple. En d'autres termes, la gaine souple telle que décrite précédemment fait partie de la housse qui protège le capteur. Ainsi, une seule pièce de tissu équipé de coutures fait office de gaine souple et de housse enveloppante la portion d'acquisition. Autrement dit, la housse et la gaine sont incarnées par une unique pièce, par exemple de tissu, qui réalise les deux fonctions, i.e. le placement et le maintien des panneaux ainsi que la protection enveloppante. En intégrant le panneau rigide ou les plaquettes rigides dans la housse, on obtient un appareil d'acquisition simple d'utilisation, formé de peu de pièces distinctes.

Selon une autre formulation, la housse peut comprendre une face supérieure, destinée à être contre le matelas et une face inférieure, destinée à être contre les éléments de sommier, la housse étant configurée pour recevoir le capteur entre la face supérieure et la face inférieure, et dans lequel la gaine souple forme la face inférieure de la housse.

Selon une option, l'appareil d'acquisition peut comprendre en outre des moyens d'attache (ou au moins une attache). Les moyens d'attache peuvent s'étendre, directement ou indirectement (par exemple à partir de la housse) à partir de la portion d'acquisition, de part et d'autre de la portion d'acquisition, notamment selon la direction transversale Y.

On note que les moyens d'attache peuvent se présenter comme des lanières équipées de surface velours crochet Velcro^{™}.

Ceci permet d'arrimer l'appareil d'acquisition à une ou plusieurs lattes. On assure ainsi un maintien en place de l'appareil même avec une inclinaison substantielle du sommier (lit médicalisé ou plus généralement lit inclinable).

Selon une option, il peut être prévu que les moyens d'attache sont fixés de manière détachable à l'appareil d'acquisition, en particulier à la housse. On a ainsi une certaine liberté pour positionner les moyens d'attache par rapport à la portion d'acquisition et aux lattes du sommier.

Selon une option, le capteur peut comprendre une chambre pneumatique. Il s'agit d'une solution bien maîtrisée, fiable et durable.

La chambre pneumatique peut être équipé de pontets. De tels pontets limitent le bombage de la chambre pneumatique lorsqu'elle est gonflée.

Selon une option, il peut être prévu en outre une unité électronique, qui permet notamment de traiter des données brutes délivrées par le capteur. L'unité électronique peut comprendre par exemple un transducteur, un conditionneur de signal, ou encore un module de couplage de communication sans fil. L'unité électronique peut être montée dans un boitier, situé à une extrémité longitudinale de l'appareil. Le boitier et le capteur sont alors l'un à proximité de l'autre le long de la direction longitudinale (X).

Selon une option, l'unité électronique peut être alimentée par une batterie électrique locale. Selon une autre option, l'appareil d'acquisition peut comprendre un câble de connexion pour être alimenté depuis une alimentation externe.

Selon une option, le capteur est un capteur pneumatique, qui peut être couplé à une pompe et une vanne de décharge. La chambre pneumatique est gonflée pour les phases d'acquisition et peut être dégonflée pour les phases de rangement. La pompe et la vanne de décharge peuvent être montées dans un boitier, par exemple le boitier logeant l'unité électronique.

Selon une option, l'appareil d'acquisition comprend un marquage visuel qui indique à l'utilisateur dans quel sens l'appareil d'acquisition s'installe. En particulier, le marqueur visuel et le dispositif de support peuvent être chacun d'un côté opposé du capteur. De la sorte, lorsque l'utilisateur installe l'appareil d'acquisition, il lui suffit de garder ledit marqueur visuel en visu pour que l'appareil soit naturellement mis dans le bon sens (le dispositif de support entre les éléments de sommier et le capteur). Le marquage visuel peut être un logo, une forme, la position du boitier (par exemple par rapport à une nappe du capteur), etc. Le marquage visuel peut indiquer la face de l'appareil d'acquisition qui doit être contre le matelas : dès lors, le dispositif de support se trouve de l'autre côté.

Selon une option, l'appareil d'acquisition peut être configuré pour être replié dans une configuration de rangement de l'appareil. L'appareil peut ainsi prendre une forme de faible encombrement général pour faciliter sa livraison, son rangement ou son stockage.

Il est aussi proposé un kit comprenant au moins tous les éléments décrits précédemment et permettant d'assembler un appareil d'acquisition tel que décrit précédemment. En particulier, il se peut que le capteur et le dispositif de support soit livré dans des boites séparées ou bien soient disposés côte à côte dans une boite. L'utilisateur doit alors agencer correctement les éléments. Lorsque l'appareil d'acquisition comprend une housse amovible qui n'intègre pas le dispositif de support, le capteur et le dispositif de support peuvent être, à la livraison, hors de la housse.

Il est aussi proposé ici un ensemble comprenant un élément de sommier et un appareil d'acquisition tel que décrit ci-dessus, dans lequel l'élément de sommier comprend deux latte ayant une longueur (L7) qui s'étend le long de la direction longitudinale (X) et espacées entre elles d'une distance inter-latte (L3) de le long de direction transversale (Y), et dans lequel la largeur (LY) du capteur est supérieure à la distance inter-latte et/ou chaque panneau rigide s'étend le long de la direction transversale (Y) sur une largeur supérieure à la distance inter-latte, préférablement supérieure à la distance inter-latte et la largeur d'une latte (voire de deux lattes).

Selon une autre formulation, il est proposé ici un appareil d'acquisition de données physiologiques d'un être vivant, configuré pour être installé dans une literie, l'appareil comprenant :
- une portion d'acquisition avec capteur destiné à capter des ondes mécaniques transitant dans une literie,
- un dispositif de support comprenant au moins un panneau rigide, configuré pour supporter le capteur,
- une housse, configurée pour envelopper au moins la portion d'acquisition.

Le dispositif de support est configuré pour, dans une configuration d'utilisation de l'appareil dans la literie, être en appui sur des éléments de sommier de sorte que l'élément de support soit entre les éléments de sommier et le capteur. Les éléments de sommier peuvent être discontinus, par exemple des lattes.

La housse comprend une face supérieure, destinée à être contre le matelas et une face inférieure, destinée à être contre les éléments de sommier, le capteur pouvant être reçu à l'intérieur de la housse entre la face supérieure et la face inférieure. En particulier, la face inférieure peut former une gaine à l'intérieur de laquelle se trouve l'au moins un panneau rigide. La gaine peut comprendre deux pans essentiellement parallèles entre eux, qui définissent entre eux un logement à l'intérieur duquel le trouve l'au moins un panneau rigide. Alternativement, la face inférieure forme une gaine qui comprend une pluralité de logements séparés, chaque logement comprenant un panneau rigide.

Les options mentionnées précédemment sont généralement applicables pour cette formulation.

Selon un aspect particulier, il est proposé le dispositif d'acquisition sans le dispositif de support susmentionné mais avec l'attache ou les attaches. Autrement dit il est proposé un appareil d'acquisition de données physiologiques d'un être vivant, configuré pour être installé dans une literie, l'appareil d'acquisition comprenant une portion d'acquisition qui comprend un capteur destiné à capter des ondes mécaniques transitant dans la literie, et des moyens d'attache qui peuvent s'étendre, directement ou indirectement (par exemple à partir de la housse) à partir de la portion d'acquisition, de part et d'autre de la portion d'acquisition, notamment selon la direction transversale (Y) pour attacher portion d'acquisition aux éléments de sommier.

### DESCRIPTIF DES FIGURES

D'autres caractéristiques, détails et avantages de l'invention apparaîtront à la lecture de la description détaillée ci-après, et à l'analyse des dessins annexés.
La figure 1 représente en vue de dessus un appareil d'acquisition installé sur un sommier à lattes, sans matelas.
La figure 2 illustre schématiquement une partie de la portion d'acquisition de l'appareil d'acquisition, avec plusieurs panneaux rigides, et des zones de pliage entre panneaux.
La figure 3 représente, en coupe verticale selon la direction longitudinale de la portion d'acquisition, un exemple de réalisation de l'appareil d'acquisition, avec des lattes de sommier.
La figure 4 illustre schématiquement en vue de dessus une configuration à six panneaux de dimensions identiques.
La figure 5 illustre schématiquement en vue de dessus une configuration à sept panneaux de largeur de plus en plus petite en allant d'une extrémité longitudinale à l'autre.
La figure 6 illustre schématiquement en vue de dessus une configuration à neuf panneaux ayant des largeurs croissantes depuis le milieu jusqu'au extrémités longitudinales.
La figure 7 représente, en coupe verticale selon la direction longitudinale de la portion d'acquisition, un autre exemple de réalisation du dispositif d'acquisition, un panneau rigide ayant été non représenté.
La figure 8 représente, en coupe verticale selon la direction longitudinale de la portion d'acquisition, un autre exemple de réalisation du dispositif d'acquisition, un panneau rigide ayant été non représenté.
La figure 9 représente, en coupe verticale selon la direction longitudinale de la portion d'acquisition, un autre exemple de réalisation du dispositif d'acquisition, un panneau rigide ayant été non représenté.
La figure 10 représente, en perspective, un ensemble de panneaux articulés les uns aux autres.
La figure 11 représente, en coupe verticale selon la direction longitudinale de la portion d'acquisition, un autre exemple de réalisation du dispositif d'acquisition, un panneau rigide ayant été non représenté.
La figure 12 illustre en vue générale en perspective un exemple de réalisation d'un appareil d'acquisition.
La figure 13 représente, en coupe verticale, uniquement la housse dans un mode de réalisation illustrée la figure 9.

### DESCRIPTION DE MODES DE REALISATION

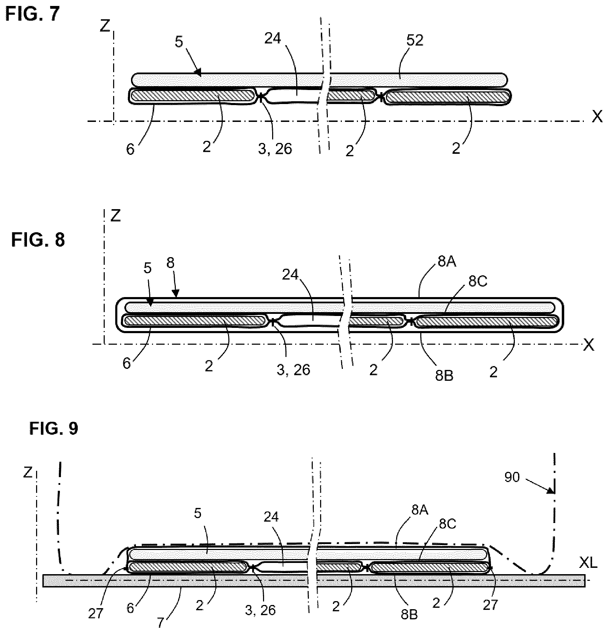

Sur les différentes figures, les mêmes références désignent des éléments identiques ou similaires. Pour des raisons de clarté de l'exposé, certains éléments ne sont pas nécessairement représentés à l'échelle. Notamment pour faciliter la compréhension, les épaisseurs et espacements selon l'axe Z ont été artificiellement augmentés aux figures 7, 8, 9 et 11.

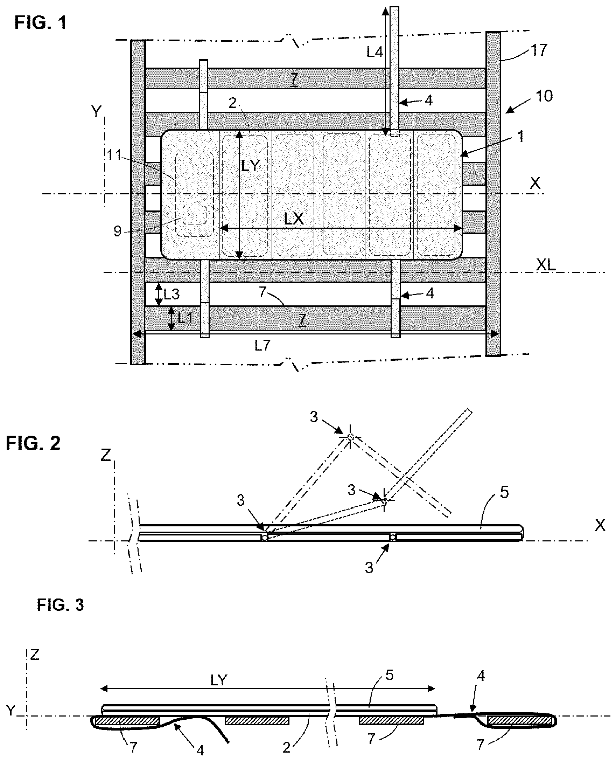

Sur la figure 1, on a représenté en partie un sommier de literie 10. Il s'agit ici d'un sommier à lattes qui comprend deux longerons latéraux 17 et des lattes 7 transversales aux longerons 17, les lattes 7 étant supportées au moins à leurs deux extrémités par les longerons 17. La dimension appelée « largeur » du lit ou largeur du sommier, repérée L7, peut être de 90 cm, 120 cm, 140 cm, 160 cm. D'autres dimensions ne sont pas exclues. On note que deux sommiers peuvent être accolés côte à côte pour les grandes largeurs de lit.

On parlera d'élément de sommier 10 pour désigner des lattes ou tout autre partie d'un sommier sur lequel le matelas prend appui.

Selon une direction dite principale du lit (appelée longueur), l'espacement entre les lattes, dit distance inter-latte, est L3 et la largeur des lattes est L1.

Un appareil d'acquisition 1 est installé sur les lattes. Un matelas 90 (représenté en traits mixtes uniquement à la figure 9) est ensuite installé au-dessus de l'appareil d'acquisition 1. On note que les caractéristiques du matelas peuvent être quelconques, par exemple quant à son épaisseur ou à sa souplesse. L'appareil d'acquisition peut fonctionner pour tout type de matelas.

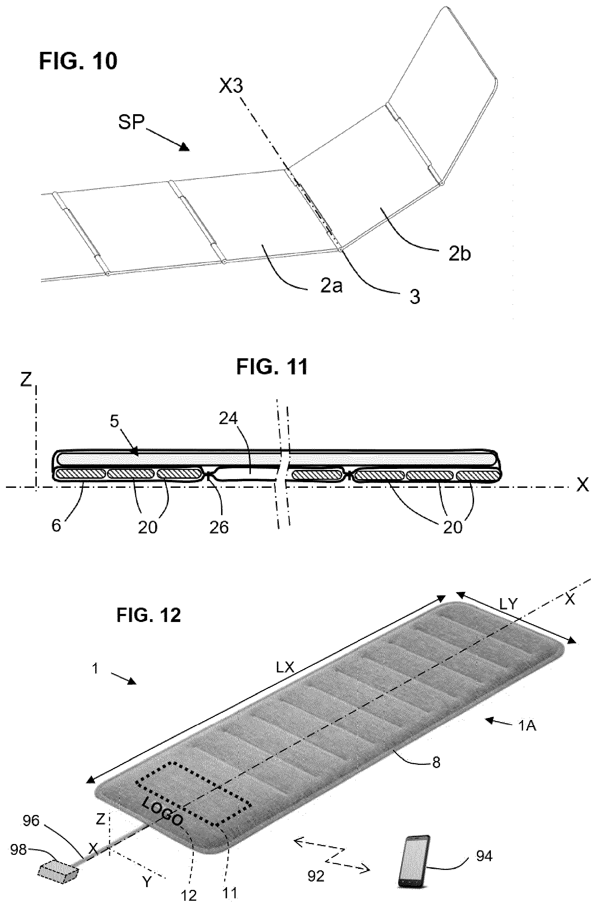

La figure 12 illustre en perspective un appareil d'acquisition selon un exemple de réalisation.

On note que dans un lit large type biplace, on peut installer deux appareils d'acquisition, par exemple un pour chaque occupant.

Le lit considéré peut être un lit domestique classique. Toutefois, l'invention concerne également les lits de type lit médicalisé. Dans les deux cas, il peut être prévu une fonction d'inclinaison partielle de l'élément de sommier.

Dans la suite, on définit une direction longitudinale X qui se comprend par rapport à la longueur de l'appareil d'acquisition 1 alors qu'une direction transversale Y s'entend par rapport à la largeur de l'appareil d'acquisition. Les directions longitudinales X et transversales Y sont typiquement orthogonales entre elles. Ce repère XY est donc différent par rapport aux directions habituelles relatives à la literie elle-même.

L'appareil d'acquisition 1 comprend une portion d'acquisition 1A capable de capter les ondes mécaniques générée par un être vivant positionné sur le matelas, comme discuté par ailleurs. La portion d'acquisition se présente comme une nappe peu épaisse. En outre, l'appareil d'acquisition 1 peut comprendre des éléments auxiliaires, par exemple des une unité électronique 9 et/ou une unité pneumatique, qui seront discutés plus loin. Les éléments auxiliaires peuvent être logés dans un boitier 11 attaché, sur une face de la nappe, à une extrémité longitudinale de celle-ci.

L'utilisateur génère des signaux biologiques mécaniques par le mouvement de ses organes internes ou par un mouvement corporel, volontaire ou réflexe. Les mouvements induits par la respiration font partie de ces mouvements, manifestation mécanique du ronflement incluse. Les mouvements induits par le battement cardiaque font partie de ces mouvements. Ces ondes transitent par le matelas 90, quelle que soit son épaisseur ou sa consistance, et sont reçues par la portion d'acquisition 1A, en particulier le capteur 5.

La portion d'acquisition 1A est peu épaisse et est, en utilisation normale, interposée entre les lattes et le matelas. La portion d'acquisition comprend un capteur 5. Le capteur peut être un capteur de mouvement ou un capteur de pression (l'un n'excluant pas nécessairement l'autre). Par exemple, le capteur peut être un capteur de type pneumatique ou de type piézoélectrique, un capteur de déformation (jauge de déformation par exemple). D'autres technologies ne sont pas exclues pour autant. Le capteur 5 est agencé dans la portion d'acquisition 1A de l'appareil d'acquisition 1.

Outre le capteur 5, la portion d'acquisition 1A comprend un dispositif de support SP, destiné à être placé en utilisation normale entre le capteur 5 et l'élément de sommier 10. Le dispositif de support SP vient directement ou indirectement au contact du capteur 5 pour lui servir d'appui.

Lorsque l'appareil d'acquisition 1 est mis en place, chaque latte 7 s'étend selon un axe XL parallèle à l'axe longitudinal X de l'appareil d'acquisition (i.e. de la portion d'acquisition). La direction transversale notée Y de la portion d'acquisition correspond à la direction longitudinale usuelle du lit.

Les lattes peuvent être simples ou double, i.e. en double épaisseur c'est-à-dire deux lattes superposées.

Les lattes peuvent être planes ou bombées. Souvent, chaque latte présente une courbure qui forme une légère bosse au milieu du sommier. Cette courbure est annulée voire inversée par une charge appliquée à la latte.

Chaque latte présente une certaine souplesse pour contribuer au confort de l'utilisateur utilisant le lit. Chaque latte 7 se déforme élastiquement sensiblement proportionnellement au poids qui est appliqué à son encontre.

L'écart L3 entre les lattes peut être du même ordre que la largeur des lattes L1. Dans certaines configurations, L3 et supérieure ou inférieure à L1. L'écartement des lattes peut être constant ou non sur l'ensemble du sommier.

La largeur des lattes L1 est dans la pratique comprise entre 5 cm et 15 cm le plus souvent autour de 10 cm.

On note LY la dimension du capteur 5 selon la direction transversale Y (aussi appelée largeur du capteur) et LX la dimension du capteur selon la direction longitudinale X.

On fait en sorte que LY soit nettement supérieure à L1 ou L3. On peut choisir par exemple la condition LY > 2 x L1, ou LY > 2 x L1 + L3, ou LY > 2x (L1 + L3) ou encore LY > 3 x L1 ou LY > 3x (L1 + L3).

Autrement dit, généralement, le capteur 5 est installé à cheval sur au moins deux lattes 7. Dans certains cas, il peut reposer sur trois lattes ou plus.

La largeur LY du capteur 5 peut être comprise entre 10 cm et 50 cm.

Généralement, la longueur LX du capteur 5 peut être comprise entre 40 cm et 60 cm.

La longueur des lattes L7 peut être dans la pratique comprise entre 80 cm et 120 cm.

Le dispositif de support SP comprend un ou plusieurs panneaux rigides 2. Dans les exemples illustrés, le nombre de panneaux rigides 2 est compris entre 6 et 10 ; toutefois un nombre quelconque rentre dans le cadre de l'invention sans exclure une configuration à un seul panneau rigide. On peut avoir entre 3 et 20 panneaux, voire entre 5 et 15 panneaux. Les panneaux rigides 2 peuvent être disposés de façon adjacente le long de la direction longitudinale X.

Dans une configuration d'utilisation normale du dispositif d'acquisition dans la literie, la portion d'acquisition 1A vient en appui sur les lattes ou plus généralement des éléments de sommier pouvant être discontinus. Au moins 80% de la surface du capteur 5 est disposée sur le dispositif de support SP, préférablement 90%.

Le dispositif de support SP est configuré, en utilisation, pour être interposé entre les éléments de sommier 10 et le capteur 5. Il fournit ainsi, même en cas d'éléments de sommier discontinus, un appui substantiellement continu au capteur recevant les ondes mécaniques générées au travers de la literie par un utilisateur allongé sur le matelas. La sensibilité du capteur aux ondes mécaniques est ainsi améliorée et ne dépend plus du type d'élément de sommier.

Avoir plusieurs panneaux rigides 2 permet d'avoir un dispositif de support SP pliable (pour le stockage ou l'emballage). A cet égard, le dispositif de support SP peut prévoir une zone de pliage 3 entre deux panneaux adjacents. La zone de pliage 3 peut autoriser une pliure d'axe parallèle à la direction transversale Y. Dans le cas où plusieurs panneaux 2 composent le dispositif de support SP, il est prévu, entre des paires de panneaux adjacents 2a,2b (cf. figure 7, 8 ou 10), une zone de pliage 3 autorisant une pliure d'axe parallèle à la direction transversale Y.

Selon un exemple, lorsque le dispositif de support comprend N panneaux, il est prévu N-1 zones de pliage, les pliures pouvant être substantiellement parallèles entre elles.

Le sens de pliage peut être le même sur toutes les zones de pliage ou bien alternativement à la façon d'un accordéon, les deux possibilités étant illustrées la figure 2. L'amplitude du pliage possible de chaque zone de pliage peut être symétrique ou non.

Dans un mode de réalisation, illustré sur la figure 10, le dispositif de support comprend une pluralité de panneaux 2a, 2b contigus et reliés entre eux par une charnière mécanique (ou articulation) faisant office de zone de pliage 3. L'articulation peut se faire le long de l'axe X3 qui est parallèle à la direction transversale Y.

Dans un mode de réalisation illustré sur les figures 7, 8 et 9, le ou les panneaux 2 sont intégrés à une gaine souple 6 (ou enveloppe). Par exemple, les panneaux peuvent être disposés les uns des autres le long de la direction longitudinale X. Les panneaux peuvent être contigus comme décrits précédemment ou bien adjacents mais non contigus. Les zones de la gaine souple dépourvues de panneaux peuvent alors servir de zones de pliage 3. La gaine souple 6 comprend notamment une face supérieure et une face inférieure.

La gaine souple peut être réalisée en tissu tissé ou non tissé. L'utilisation d'un film de matière plastique polymère peut être envisagé. L'utilisation d'un tissu à base de fibres naturelles biodégradables est aussi considérée. La gaine 6 peut être un tissu continu ou un tissu maillé ou encore un filet. Localement, l'épaisseur de la gaine est très faible, le tissu ou équivalent présente une souplesse importante à la flexion ; on peut avoir aussi une certaine élasticité longitudinale du matériau.

La gaine souple 6 peut former un unique logement qui reçoit le panneau ou la pluralité de panneaux. Alternativement, la gaine souple 6 comprend une pluralité de logements 24, typiquement séparés par une zone de jonction 26. Les panneaux sont insérés dans les logements. Selon une configuration, il y a un panneau par logement. Les logements 24 peuvent être agencés les uns à côté des autres le long de la direction longitudinale (X), séparés entre eux par au moins une zone de jonction 26, voire par une zone de jonction 26 entre chaque paire de panneaux 2 adjacents. La zone de jonction 26 fait office de zone de pliage 3, qui peut être orthogonale à la direction longitudinale.

Pour réaliser la zone de jonction 26, la face supérieure et la face inférieure de la gaine souple 6 peuvent être jointes localement pour ainsi former les logements. Plus précisément, une couture relie la face supérieure de la gaine à la face inférieure de la gaine dans la zone de jonction 26. Ceci assure un espacement entre les deux panneaux adjacents qui permet un pliage d'amplitude importante, voire quasiment une superposition de deux panneaux adjacents (par exemple supérieur à 160°).

Dans certaines réalisations, le dispositif d'acquisition 1 peut comprendre en outre une housse 8 enveloppant au moins la portion d'acquisition 1A.

Ladite housse 8 peut réaliser également la fonction gaine souple ou peut être totalement distincte de la gaine souple indépendante, comme ceci est illustré à la figure 8. La gaine est alors logée à l'intérieur de la housse, de sorte à être positionnée entre les éléments de sommier et le capteur.

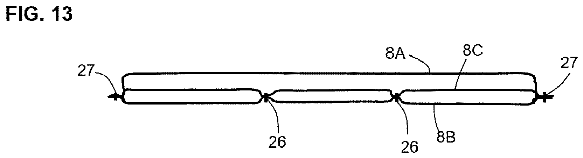

Dans une configuration où la housse fait office de gaine, comme illustré aux figures 9 et 13, la housse 8 comprend une face supérieure 8A, destinée à être contre le matelas et une face inférieure 8B, 8C, destinée à être contre les éléments de sommier. Le capteur est alors reçu à l'intérieur de la housse entre la face supérieure et la face inférieure.

Dans un mode de réalisation, la face inférieure comprend la gaine telle que décrite précédemment.

A cette fin, une pièce de tissu est disposée en trois pans sur une surface correspondant au moins à la surface du capteur.

Le pan supérieur 8A est destiné à recouvrir au moins le capteur 5. Le pan inférieur 8B est destiné à être interposé entre les panneaux du dispositif de support SP et les éléments de sommier. Le pan intermédiaire 8C est interposée entre le capteur 5 et les panneaux du dispositif de support. On peut pratiquer des coutures de liaison 27 sur la bordure extérieure. On peut pratiquer des coutures intermédiaires 26 pour délimiter et former des logements recevant les panneaux 2.

Dans une configuration alternative illustrée la figure 11, il peut y avoir plusieurs panneaux ou plaquettes dans chaque logement. Les plaquettes d'un même logement peuvent voir leur position relative évoluer par exemple pour un enroulement complet façon escargot de la portion d'acquisition.

Chaque panneau présente dans les exemples une forme générale rectangulaire, de faible épaisseur (par exemple comprise entre 1 à 3 mm).

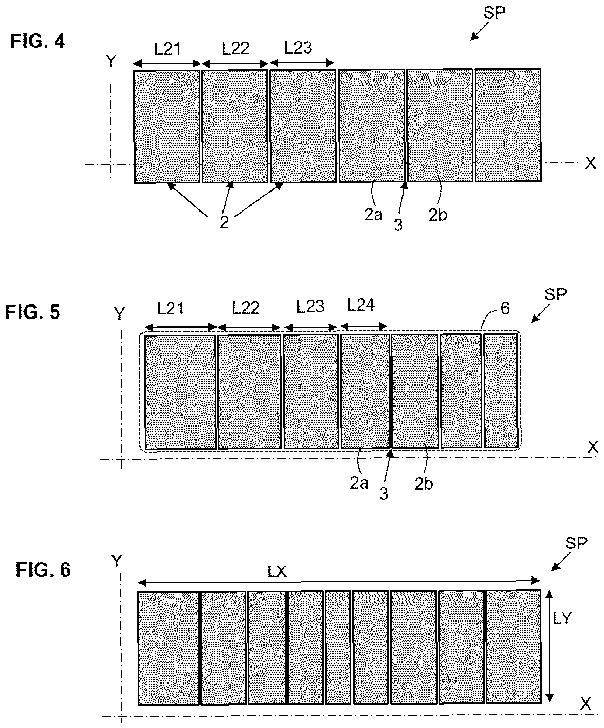

Concernant la dimension de chaque panneau le long de l'axe longitudinal X, selon un exemple de réalisation illustré à la figure 4, tous les panneaux peuvent avoir la même dimension L21=L22=L23 comme illustré à la figure 4. La largeur des panneaux L2i=L21 peut être comprise entre 3 cm et 10 cm. On pourrait choisir L2i = 4 cm ou L2i = 5 cm.

Les dimensions des panneaux le long de l'axe transversal Y sont sensiblement équivalentes à la largeur du capteur LY, de sorte que substantiellement tout le capteur repose sur le dispositif de support. Par sensiblement équivalentes ou substantiellement, on entend que ladite dimension des panneaux est égale à au moins 80%, voire 90% ou encore 95%, de la largeur du capteur LY.

Selon un autre exemple illustré à la figure 5, la largeur L2i est de plus en plus petite en allant d'une extrémité longitudinale à l'autre : L21>L22>L23 etc....

Selon un autre exemple illustré à la figure 6, la largeur L2i est minimale au milieu et les panneaux sont de plus en plus large en allant vers les extrémités.

On peut utiliser tout type de matériau plastique pour réaliser les panneaux rigides par exemple du PVC, du polyéthylène, du polypropylène, de l'ABS. L'utilisation d'un matériau naturel tel que du bois est aussi possible.

Pour placer l'appareil d'acquisition 1, l'utilisateur peut être guidé par un ou plusieurs marqueurs visuels 12 (voir 'LOGO' sur la figure 12) disposés sur l'appareil d'acquisition 1. Typiquement, dans le cas d'une housse, le ou les marqueurs sont disposés sur la housse. Le marqueur 12 peut être une marque, un logo, ou tout signe visuellement différenciant les deux faces de l'appareil d'acquisition, comme la position du boitier par rapport à la nappe du capteur ou les coutures sur la housse. Typiquement, le marqueur visuel 12 et le dispositif de support SP sont positionnés de part et d'autre du capteur 5, de sorte que si l'utilisateur maintient un contact visuel avec le marqueur 12 pendant l'installation, il est assuré que l'appareil d'acquisition 1 est positionné dans le bon sens.

Comme illustré aux figures 1 et 3, le dispositif d'acquisition 1 peut comprendre en outre au moins une attache 4 (appelée par la suite moyens d'attache) s'étendant à partir de la portion d'acquisition de part et d'autre de la portion d'acquisition. Dans l'exemple illustré, ces moyens d'attache 4 se présentent comme des lanières de rétention.

Dans une variante, le dispositif d'acquisition 1 ne comprend pas le dispositif de support mais comprend l'attache 4.

Sur la figure 1, la lanière représentée en haut à droite n'est pas attachée et est représentée dans une configuration rectiligne. Dans l'exemple illustré, l'appareil d'acquisition est équipé de quatre lanières, mais une configuration à deux lanières ou trois lanières est aussi possible.

Dans l'exemple illustré, les moyens d'attache sont formés comme des lanières de tissu ou des bandes de tissus. La matière peut être un plastique polymère ou un tissu de fibres synthétiques ou naturelles.

La longueur de ces bandes L4 est suffisante pour faire le tour d'une latte, voire le tour d'une latte et deux fois la largeur d'intervalle L3 entre les lattes.

Dans un exemple, la longueur des lanières L4 est comprise entre 30 cm et 60 cm.

Dans l'exemple illustré, il est prévu d'agencer sur une partie de la lanière une zone de crochet velcros. Sur une autre portion de la lanière, il est prévu une zone de velours velcros de sorte que la lanière peut être attachée à elle-même et son parcours permet d'enrouler au moins une latte 7. Autrement dit, dans le cas général, la lanière fait une boucle autour d'une latte et revient s'attacher à elle-même.

S'agissant des parcours possibles de la lanière, en référence à la figure 3, on remarque que l'extrémité de la lanière peut être raccrochée à une portion intermédiaire de la lanière (cf. partie droite figure 3) ; une autre possibilité est qu'une zone d'extrémité de la lanière soit raccrochée à la housse (cf. partie gauche figure 3).

Selon une option, chacune des lanières 4 est cousue à la housse 8.

Selon une autre option, chacune des lanières est associée de manière détachable à la housse 8. Par exemple ici aussi on peut utiliser un système de type Velcro^{™} pour attacher la lanière à une zone de bordure de la housse.

Selon une option, non représenté aux figures, il peut être prévu une lanière plus longue, qui s'étend part et d'autre de la portion d'acquisition de longueur voisine de 2xL3 + LY ; grâce à quoi une seule lanière peut faire un parcours de boucle de chaque côté de la portion d'acquisition. Avec deux lanières longues, on fournit quatre zones d'attache sur les lattes. Bien entendu les lanières seront repliées sur la portion d'acquisition lorsque l'on veut ranger l'appareil d'acquisition.

Dans une réalisation alternative non représentée aux figures, il peut être prévu des lacets comme moyens d'attache.

Dans une réalisation alternative non représentée aux figures, il peut être prévu sur chaque lanière une boucle de verrouillage de type ceinture de vêtement.

Comme mentionné précédemment, l'appareil d'acquisition peut comprendre en outre une unité électronique 9, qui peut être logé dans un boitier 11. Dans cette unité électronique, il peut être prévu un circuit de mise en forme et/ou de traitement des données brutes délivrées par le capteur. Il peut être prévu également un coupleur de communication sans fil. L'appareil d'acquisition peut en outre comprendre une batterie d'alimentation électrique locale.

Dans le cas où le capteur est un capteur de type pneumatique, le capteur se présente comme une chambre pneumatique gonflable. Alors l'appareil d'acquisition peut en outre comprendre une pompe pneumatique miniature 19 pour gonfler la chambre pneumatique, et une vanne de décharge pour purger la chambre pneumatique. La pompe 19 peut être logé dans le boitier 11 aussi.

Comme visible sur la figure 12, l'appareil d'acquisition peut comprendre un câble 96, notamment pour l'alimentation électrique de l'appareil. L'extrémité du câble est équipée d'un connecteur 98 (par exemple micro-USB).

Concernant la transmission de données, l'appareil d'acquisition peut être équipé dans son unité électronique d'un coupleur de communication sans fil 92 configuré pour émettre voire émettre et recevoir des données en coopération avec une unité distante 94 (smartphone ou serveur distant). Des protocoles BlueTooth ou BlueTooh Low Emission, et/ou Wi-Fi peuvent être utilisés.

Le boitier 11 peut être logé à côté de la portion d'acquisition 1A, le long de la direction longitudinale X. Par exemple, le boitier peut être monté sur la nappe formant le capteur 5. La housse 8 est alors dimensionnée de façon à accueillir le boitier et la portion d'acquisition.

L'appareil d'acquisition peut avoir une longueur comprise entre 50cm et 1m, voire entre 50 et 80cm, voire entre 60 et 70cm. L'appareil d'acquisition peut avoir une largeur comprise entre 10 et 30cm, voire entre 15 et 25 cm, voire entre 18 et 23cm. La housse définissant l'extérieur de l'appareil d'acquisition, elle présente les mêmes dimensions. La forme générale de l'appareil d'acquisition peut être rectangulaire, avec une épaisseur comprise entre 0,4cm et 2cm, voire entre 0,4cm et 1cm, voire aux alentours de 5mm. La masse de l'appareil d'acquisition peut être inférieur à 500g.

## Revendications

1. Appareil d'acquisition de données physiologiques d'un être vivant, configuré pour être installé dans une literie avec des éléments de sommier, l'appareil comprenant une portion d'acquisition (1A) qui comprend :
- un capteur (5) destiné à capter des ondes mécaniques transitant dans la literie,
- un dispositif de support (SP) comprenant une pluralité de panneaux rigides (2), configuré pour supporter le capteur,
le dispositif de support (SP) étant configuré pour, dans une configuration d'utilisation de l'appareil d'acquisition dans la literie, être en appui sur des éléments de sommier, de sorte que le dispositif de support (SP) soit interposé entre les éléments de sommier et le capteur.
dans lequel la portion d'acquisition (1A) s'étend selon une direction longitudinale (X) et la pluralité de panneaux (2) rigides sont agencés les uns à côté des autres selon l'axe longitudinal (X) de la portion d'acquisition, reliés entre eux par une zone de pliage (3) autorisant un pliage du dispositif de support (SP).

2. Appareil d'acquisition selon la revendication 1, comprenant en outre une unité électronique (9) configurée pour mettre en forme et/ou traiter des données brutes délivrées par le capteur (5).

3. Appareil d'acquisition selon la revendication 1 ou 2, dans lequel les panneaux sont intégrés à une gaine souple (6), qui s'étend le long de la direction longitudinale (X), les zones de la gaine souple dépourvues de panneaux formant des zones de pliage (3).

4. Appareil d'acquisition selon la revendication 3, dans lequel la gaine souple forme une pluralité de logements (24), les panneaux étant insérés dans les logements, par exemple un panneau par logement.

5. Appareil selon l'une quelconque des revendications 1 à 4, dans lequel chaque panneau présente une largeur selon un axe transversal (Y) orthogonal à l'axe longitudinal (X) comprise entre 3 cm et 10 cm.

6. Appareil selon l'une quelconque des revendications 1 à 5, dans lequel l'appareil d'acquisition est configuré pour être replié dans une configuration de rangement de l'appareil.

7. Appareil selon la revendication 6, dans lequel le capteur comprend une chambre pneumatique, couplé à une pompe et une vanne de décharge, la chambre pneumatique étant configuré pour être gonflée dans la configuration d'utilisation et à être dégonflée dans la configuration de rangement.

8. Appareil d'acquisition selon l'une des revendications 1 à 7, dans lequel le capteur présente une largeur de capteur (LY) le long d'une direction transversale (Y) et chaque panneau rigide s'étend, le long de la direction transversale (Y), sur au moins 80% de la largeur du capteur, voire 90% de la largeur du capteur.

9. Appareil d'acquisition selon l'une des revendications 1 à 8, comprenant en outre une housse (8) enveloppant au moins la portion d'acquisition, la housse étant de préférence amovible.

10. Appareil d'acquisition selon les revendications 4 et 9, dans lequel la housse comprend une face supérieure (8A), destinée à être contre le matelas et une face inférieure (8B), destinée à être contre les éléments de sommier, la house étant configurée pour recevoir le capteur entre la face supérieure et la face inférieure, et dans lequel la gaine souple forme la face inférieure de la housse.

11. Appareil d'acquisition selon l'une des revendications 1 à 10, comprenant en outre au moins une attache, l'attache étant configurée pour être attachée aux éléments de sommier.

12. Appareil d'acquisition selon l'une quelconque des revendications 1 à 11, dans lequel au moins un panneau parmi l'au moins un panneau est plein ou ajouré.

13. Appareil d'acquisition selon l'une quelconque des revendications 1 à 12, comprenant un marqueur visuel, par exemple un logo, indiquant à l'utilisateur le sens d'utilisation de l'appareil d'acquisition, le marqueur visuel et le dispositif de support (SP) étant chacun d'un côté opposé du capteur.

14. Kit pour assembler un appareil selon l'une quelconque des revendications 1 à 13, le kit comprenant le capteur et le dispositif de support (SP).

15. Ensemble comprenant un élément de sommier et un appareil d'acquisition selon l'une quelconque des revendications 1 à 13, dans lequel l'élément de sommier comprend deux latte ayant une longueur (L7) qui s'étend le long de la direction longitudinale (X) et espacées d'une distance inter-lattes (L3) de le long de direction transversale (Y), et dans lequel la largeur (LY) du capteur est supérieure la distance inter-latte et/ou chaque panneau rigide s'étend le long de la direction transversale (Y) sur une largeur supérieure à la distance inter-lattes (L3).

## Patentansprüche

1. Gerät zum Erfassen physiologischer Daten eines Lebewesens, die so konfiguriert ist, dass sie in ein Bettzeug mit Bettfederelementen eingebaut werden kann, wobei die Vorrichtung einen Erfassungsabschnitt (1A) umfasst, der Folgendes umfasst:
- einen Sensor (5), der dazu bestimmt ist, mechanische Wellen zu erfassen, die durch die Bettwäsche hindurchgehen,
- eine Stützvorrichtung (SP), die eine Vielzahl von starren Platten (2) umfasst und so konfiguriert ist, dass sie den Sensor stützt,
die Stützvorrichtung (SP) so konfiguriert ist, dass sie in einer Konfiguration für die Verwendung des Erfassungsgeräts im Bettzeug auf Lattenrostelementen aufliegt, so dass die Stützvorrichtung (SP) zwischen den Lattenrostelementen und dem Sensor angeordnet ist.
wobei sich der Erfassungsabschnitt (1A) in einer Längsrichtung (X) erstreckt und die Vielzahl von starren Platten (2) nebeneinander entlang der Längsachse (X) des Erfassungsabschnitts angeordnet sind, die durch einen Faltbereich (3) miteinander verbunden sind, der ein Falten der Stützvorrichtung (SP) zulässt.

2. Erfassungsgerät nach Anspruch 1, das außerdem eine elektronische Einheit (9) umfasst, die so konfiguriert ist, dass sie die vom Sensor (5) gelieferten Rohdaten aufbereitet und/oder verarbeitet.

3. Erfassungsgerät nach Anspruch 1 oder 2, bei dem die Paneele in eine flexible Hülle (6) integriert sind, die sich entlang der Längsrichtung (X) erstreckt, wobei die Bereiche der flexiblen Hülle, die frei von Paneelen sind, Faltbereiche (3) bilden

4. Erfassungsgerät nach Anspruch 3, bei dem die flexible Hülle eine Vielzahl von Aufnahmen (24) bildet, wobei die Platten in die Aufnahmen eingesetzt werden, z. B. eine Platte pro Aufnahme.

5. Gerät nach einem der Ansprüche 1 bis 4, wobei jede Platte eine Breite entlang einer Querachse (Y) orthogonal zur Längsachse (X) zwischen 3 cm und 10 cm aufweist.

6. Gerät nach einem der Ansprüche 1 bis 5, wobei das Erfassungsgerät so konfiguriert ist, dass es in eine Konfiguration zur Aufbewahrung des Geräts zusammengefaltet werden kann.

7. Gerät nach Anspruch 6, wobei der Sensor eine pneumatische Kammer umfasst, die mit einer Pumpe und einem Ablassventil gekoppelt ist, wobei die pneumatische Kammer so konfiguriert ist, dass sie in der Verwendungskonfiguration aufgeblasen wird und in der Aufbewahrungskonfiguration entleert wird.

8. Erfassungsgerät nach einem der Ansprüche 1 bis 7, wobei der Sensor eine Sensorbreite (LY) entlang einer Querrichtung (Y) aufweist und sich jede starre Platte entlang der Querrichtung (Y) über mindestens 80% der Sensorbreite oder sogar 90% der Sensorbreite erstreckt.

9. Erfassungsgerät nach einem der Ansprüche 1 bis 8, ferner umfassend eine Hülle (8), die zumindest den Erfassungsabschnitt umhüllt, wobei die Hülle vorzugsweise abnehmbar ist.

10. Erfassungsgerät nach Anspruch 4 und 9, wobei der Bezug eine Oberseite (8A), die an der Matratze anliegen soll, und eine Unterseite (8B), die an den Bettkastenelementen anliegen soll, umfasst, wobei der Bezug so gestaltet ist, dass er den Sensor zwischen der Oberseite und der Unterseite aufnimmt, und wobei die flexible Hülle die Unterseite des Bezugs bildet.

11. Erfassungsgerät nach einem der Ansprüche 1 bis 10, das außerdem mindestens eine Klammer umfasst, wobei die Klammer so konfiguriert ist, dass sie an den Bettrahmenelementen befestigt werden kann

12. Erfassungsgerät nach einem der Ansprüche 1 bis 11, wobei mindestens eines der mindestens einen Paneele massiv oder durchbrochen ist.

13. Erfassungsgerät nach einem der Ansprüche 1 bis 12, mit einer visuellen Markierung, z. B. einem Logo, die dem Benutzer die Richtung der Verwendung des Erfassungsgeräts anzeigt, wobei sich die visuelle Markierung und die Stützvorrichtung (SP) jeweils auf einer gegenüberliegenden Seite des Sensors befinden.

14. Bausatz zum Zusammenbau eines Geräts nach einem der Ansprüche 1 bis 13, wobei der Bausatz den Sensor und die Haltevorrichtung (SP) umfasst.

15. Anordnung mit einem Lattenrost und einem Erfassungsgerät nach einem der Ansprüche 1 bis 13, wobei der Lattenrost zwei Latten mit einer Länge (L7) aufweist, die sich entlang der Längsrichtung (X) erstreckt und die durch einen Zwischenlattenabstand (L3) entlang der Querrichtung (Y) voneinander beabstandet sind, und wobei die Breite (LY) des Sensors größer ist als der Abstand zwischen den Latten und/oder jede starre Platte sich entlang der Querrichtung (Y) über eine Breite erstreckt, die größer ist als der Abstand zwischen den Latten (L3).

## Claims

1. An apparatus acquiring physiological data from a living being, configured to be installed in bedding with bed frame elements, the apparatus comprising an acquisition portion (1A) which comprises:
- a sensor (5) designed to pick up mechanical waves passing through the bedding,
- a support device (SP) comprising a plurality of rigid panels (2), configured to support the sensor,
the support device (SP) being configured to rest on bed frame elements in a configuration for use of the acquisition device in bedding, so that the support device (SP) is interposed between the bed frame elements and the sensor.
wherein the acquisition portion (1A) extends along a longitudinal direction (X) and the plurality of rigid panels (2) are arranged side by side along the longitudinal axis (X) of the acquisition portion, connected to each other by a folding zone (3) allowing folding of the support device (SP).

2. The acquisition device according to claim 1, further comprising an electronic unit (9) configured to format and/or process raw data delivered by the sensor (5).

3. The acquisition device according to claim 1 or 2, wherein the panels are integrated into a flexible sheath (6), which extends along the longitudinal direction (X), the areas of the flexible sheath devoid of panels forming folding areas (3).

4. The acquisition device according to claim 3, wherein the flexible sheath forms a plurality of housings (24), the panels being inserted into the housings, for example one panel per housing.

5. The device according to any one of claims 1 to 4, wherein each panel has a width along a transverse axis (Y) orthogonal to the longitudinal axis (X) of between 3 cm and 10 cm.

6. The device according to any one of claims 1 to 5, wherein the acquisition device is configured to be folded into a device storage configuration.

7. The device according to claim 6, wherein the sensor comprises a pneumatic chamber, coupled to a pump and a discharge valve, the pneumatic chamber being configured to be inflated in the use configuration and to be deflated in the storage configuration.

8. The acquisition device according to one of claims 1 to 7, wherein the sensor has a sensor width (LY) a transverse direction (Y) and each rigid panel extends, along the transverse direction (Y), over at least 80% of the sensor width, or even 90% of the sensor width.

9. The acquisition device according to one of claims 1 to 8, further comprising a cover (8) enveloping at least the acquisition portion, the cover preferably being removable.

10. The acquisition device according to claims 4 and 9, wherein the cover comprises an upper face (8A), intended to be against the mattress and a lower face (8B), intended to be against the bed frame elements, the house being configured to receive the sensor between the upper face and the lower face, and wherein the flexible sheath forms the lower face of the cover.

11. The acquisition device according to one of claims 1 to 10, further comprising at least one fastener, the fastener being configured to be attached to bed frame elements.

12. The acquisition device according to any one of claims 1 to 11, wherein at least one of the at least one panels is solid or perforated.

13. The acquisition device according to any one of claims 1 to 12, comprising a visual marker, for example a logo, indicating to the user the direction of use of the acquisition device, the visual marker and the support device (SP) each being on opposite sides of the sensor.

14. A kit for assembling a device according to any one of claims 1 to 13, the kit comprising the sensor and the support device (SP).

15. An assembly comprising a bed frame element and an acquisition device according to any one of claims 1 to 13, wherein the bed frame element comprises two slats having a length (L7) extending along the longitudinal direction (X) and spaced apart by an inter-slat distance (L3) along the transverse direction (Y), and wherein the width (LY) of the sensor is greater than the lath spacing and/or each rigid panel extends along the transverse direction (Y) over a width greater than the lath spacing (L3).
